# EUROPÄISCHE PATENTSCHRIFT

(11) **EP 1 259 195 B1**
(45) Veröffentlichungstag und Bekanntmachung des Hinweises auf die Patenterteilung: **15.10.2008**
(21) Anmeldenummer: 01929231.7
(22) Anmeldetag: 28.02.2001
(51) Int. Cl.: A61F 2/24

(54) **VORRICHTUNG ZUR BEFESTIGUNG UND VERANKERUNG VON HERZKLAPPENPROTHESEN**
DEVICE FOR FASTENING AND ANCHORING CARDIAC VALVE PROSTHESES
DISPOSITIF POUR FIXER ET ANCRER DES PROTHESES DE VALVULES CARDIAQUES

(30) Priorität: 28.02.2000 DE 10010074
(43) Veröffentlichungstag der Anmeldung: 27.11.2002
(60) Teilanmeldung: 08163041.0
(73) Patentinhaber: JenaValve Technology Inc., Wilmington, DE 19801 (US)
(72) Erfinder: WEBER, Carsten, 07743 Jena (DE); PESCHEL, Thomas, 07743 Jena (DE); DAMM, Christoph, 07743 Jena (DE); FIGULLA, Hans-Reiner, 07749 Jena (DE); FERRARI, Markus, 07747 Jena (DE)
(74) Vertreter: Trinks, Ole
(86) Internationale Anmeldenummer: PCT/DE2001/000837
(87) Internationale Veröffentlichungsnummer: WO 2001/062189

(56) Entgegenhaltungen:
- US-A- 3 755 823
- US-A- 5 411 552
- US-A- 5 855 601

## Beschreibung

Die Erfindung betrifft eine Vorrichtung zur Befestigung und Verankerung von Herzklappenprothesen, die im wesentlichen aus drahtförmigen miteinander verbundenen Elementen gebildet ist. Sie kann in zusammengefalteten Zustand minimal invasiv durch die Aorta eingeführt und in der Aortenwand nach Auffaltung verankert werden, so dass die implantierte und befestigte Herzklappenprothese die Funktion der körpereigenen Herzklappe übernehmen kann.

Bisher ist es in nicht ausreichendem Masse gelungen, eine Lösung vorzuschlagen, mit der sowohl eine sichere Abdichtung gegen die Aortenwand, wie auch ein sicherer Halt gewährleistet werden kann. Dabei muß eine solche Vorrichtung bzw. eine solche Verankerungsstütze (Stent) ausreichend klein zusammengefaltet werden können, um dann am Implantationsort aufgespannt zu werden. Bei den bekannten Lösungen wird aber keine ausreichende Vergrößerung mit entsprechender Spannkraft, die den Halt gewährleisten kann, erreicht. Auch Vorschläge bei denen ein Formgedächtnismetall (Memorymetall) verwendet werden soll, genügen nicht den Anforderungen, obwohl mit diesen Materialien bei Erreichen bzw. Überschreiten einer Sprungtemperatur eine Ausdehnung auftritt.

Auch die in US 5,411,552 beschriebene Lösung kann die Erfordernisse nicht erfüllen, da ein relativ labiles Gebilde verwendet werden soll.

In US 5,855,601 ist ebenfalls eine Herzklappenprothese beschrieben, die als relativ stabiler Stent ausgebildet ist.

Das US 3,755,823 offenbart einen flexiblen Stent für Herzklappen gemäß dem Oberbegriff des Anspruchs 1.

Ein weiteres bisher nur unbefriedigend gelöstes Problem ist die sichere Befestigung einer künstlichen oder biolgischen Herzklappenprothese. In der Regel werden die Prothesen aufwendig an einen Stent angenäht. Dies ist zeitaufwendig und muß mit großer Sorgfalt erfolgen, um Beschädigungen zu vermeiden.

Da die implantierten Herzklappenprothesen über große Zeiträume funktionstüchtig sein müssen, spielt auch die konstruktive Ausbildung eine wesentliche Rolle, da es ansonsten nach der Implantation zu Beschädigungen und Undichtheiten kommen kann, die zu lebensbedrohlichen Zuständen des Patienten führen können.

Es ist daher Aufgabe der Erfindung eine Vorrichtung zur Befestigung und Verankerung von Herzklappenprothesen vorzuschlagen, die für eine minimal invasive Implantion durch die Aorta klein zusammengefaltet und am Implantationsort entfaltet werden kann, wobei ein sicherer Halt und eine sichere Abdichtung gegenüber der Aortenwand gewährleistet ist.

Erfindungsgemäß wird diese Aufgabe mit einer Vorrichtung nach Anspruch 1 gelöst.

Vorteilhafte Ausgestaltungsformen und Weiterbildungen der Erfindung können mit den in den untergeordneten Ansprüchen genannten Merkmalen erreicht werden.

Wesentliche Elemente der erfindungsgemässen Lösung sind jeweils drei gleiche Paare von Bügeln, die in jeweils um 120° versetzter Anordnung miteinander verbunden sind. Die beiden Bügel eines Paares sind entgegengesetzt zueinander gekrümmt gebogen und mittels Festkörpergelenken verbunden. Die Festkörpergelenke erfüllen gleichzeitig die Funktion von Drehlagern, um die die Bügel eines Paares, ähnlich wie bei einer wippe, verschwenkt werden können. Wird auf einen der Bügel eine Druckkraft, z.B. durch Perestaltik der Aorta ausgeübt, wird dieser Bügel entsprechend in die gleiche Richtung um die Drehachse am Festkörpergelenk verschwenkt. Gleichzeitig wird der jeweils andere Bügel des Paares entgegengesetzt dazu verschwenkt. Demzufolge wird dann immer einer der beiden Bügel gegen die Aortenwand gedrückt, was die Abdichtung und den Halt erhöht.

Günstig ist es, die Bügel eines Paares so zu dimensionieren, dass möglichst gleiche Hebelverhältnisse in Bezug zu den die Drehlager bildenden Festkörpergelenken eingehalten werden, also gleich lange zumindest annähernd gleich lange Hebelarme gebildet werden.

Vorteilhaft sind auch die durch die 120 ° Anordnung der Bügelpaare vorgegebenen relativ großen Abstände der Festkörpergelenke und die ebenfalls von den Bügeln abgedeckten großen Flächenbereiche.

Wobei die distalen Bügel nicht nur zur Befestigung der Herzklappenprothese dienen, sondern auch Stützfunktion übernehmen.

Durch einen weiteren in distaler Richtung angeordneten und gekrümmten Bügel können die genannten Vorteile noch verbessert werden.

Dabei ist der zweite distale Bügel in seinem distalen Bereich annähernd, wie der erste distale Bügel gekrümmt ausgebildet. Teilweise sind diese beiden Bügel so ausgebildet und geformt, dass sie nebeneinander verlaufen und zwischen ihnen Spalte ausgebildet sind. Sie können an der gleichen Stelle, an dem auch die Festkörpergelenke, als Verbindung zum in proximaler Richtung gekrümmten Bügel angeordnet sind, miteinander verbunden sein. Die gebildeten Spalte sind daher in distaler Richtung offen und es können Teile der Herzklappenprothese in die Spalte eingeführt und gehalten werden.

Zumindest ein Teil eines distalen Bügels ist proximal eingezogen und bis zu einem Wendepunkt geführt in dem benachbarte Bügel zusammengeführt sind. Bei zwei distal angeordneten Bügeln trifft dies auf den jeweils distal äußeren Bügel zu.

Zur Versteifung und für eine weitere Befestigungsmöglichkeit der Herzklappenprothese kann ein winkelförmig gekrümmter, ebenfalls proximal eingezogener Bügel verwendet werden, dessen gekrümmte Teile zwischen jeweils benachbarten Bügeln angeordnet und der jeweiligen Krümmung teilweise folgend ausgebildet sind. Diese Bügel sind mit ihren jeweils distalen Enden an dem einen distal äußeren oder dem jeweils distal äußeren Bügel befestigt. Auch hier bildet die Befestigung jeweils ein Festkörpergelenk. Diese sollten jedoch in einem Abstand zu den anderen, die Bügel eines Paares verbindenden Festkörpergelenken angeordnet sein.

Bei implantierter, aufgespannter Vorrichtung können dann die Taschen einer Herzklappenprothese dort eingeschoben, gehalten und gestützt werden.

Die Bügelkonstruktion der erfindungsgemäßen Vorrichtung stützt eine Herzklappenprothese großflächig und demzufolge schonend ab. Außerdem kann sie mit wesentlich geringerem Aufwand, beispielsweise durch Nähen befestigt werden.

Die konstruktive Lösung ermöglicht einen sicheren Halt und die erforderliche Abdichtung an bzw. gegenüber der Aortenwand. Für die Abdichtung und eine verringerte Belastung für die Herzklappenprothese wirkt sich auch das Anpressen der Herzklappenprothese von innen mittels der Bügel günstig aus.

Die erfindungsgemäße Vorrichtung kann mit einem Ballonkatheter implantiert und am Implantationsort entfaltet werden. Vorteilhaft wird für die Vorrichtung ebenfalls ein Formgedächtnismetall mit geeigneter Sprungtemperatur verwendet, mit dem zusätzlich eine Dehnung erreicht werden kann. Hierfür kann eine Nikel- und Titan enthaltende Legierung, die unter der Bezeichnung Nitinol verfügbar ist, verwendet werden.

Außerdem kann der die Herzklappenprothese tragende und haltende Teil der Vorrichtung separat zu einem Stützkörper, auf den nachfolgend, bei der Beschreibung eines Ausführungsbeispieles noch Bezug genommen wird, implantiert werden, ohne dass die vorteilhaften Eigenschaften vermindert werden. Die Implantation dieses im wesentlichen aus den drei Segmenten mit daran befestigter Herzklappenprothese bestehenden Teiles, kann dann in herkömmlicher Form operativ erfolgen.

Nachfolgend soll die Erfindung an einem Ausführungsbeispiel näher erläutert werden.

Dabei zeigt:

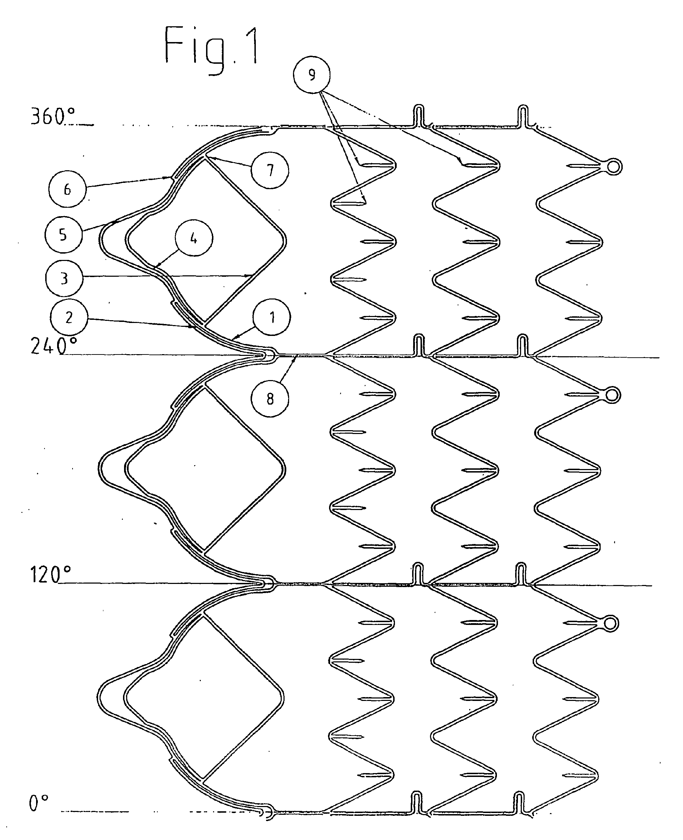

- Figur 1: eine Abwicklung eines Beispiels einer erfindungsgemäßen Vorrichtung.

In der Figur 1 ist eine Abwicklung eines Beispiels einer erfindungsgemäßen Vorrichtung gezeigt. Die Vorrichtung ist radial symmetrisch ausgebildet, wobei drei gleiche Teile in einer 120° Anordnung verwendet werden.

Jedes Teil verwendet eine Bügelkonstruktion als Träger und zur Befestigung einer künstlichen oder biologischen Herzklappenprothese.

Bei diesem Beispiel werden zwei Bügel 4 und 5 verwendet, die distal außen angeordnet sind, wobei ggf. auf den äußeren Bügel 5 verzichtet werden könnte.

Der gekrümmt gebogene Bügel 4 ist mit einem in entgegengesetzter Richtung gebogenen Bügel 3 verbunden. Die beidseitigen Verbindungen bilden Festkörpergelenke 7, die gleichzeitig Drehlagerfunktion für die beiden Hebel darstellenden Bügel 3 und 4 übernehmen, wie bereits im allgemeinen Teil der Beschreibung erklärt.

Der zweite nach außen gebogene und distal angeordnete Bügel 3 erhöht die Stabilität und bietet eine zusätzliche Abstütz- und Befestigungsmöglichkeit für die Herzklappenprothese. Dabei sind die beiden distal äußeren Bügel 4 und 5 ebenfalls miteinander verbunden, wobei die Verbindung an gleicher Stelle, an dem auch die Festkörpergelenke 7 angeordnet sind, erfolgen kann.

Zwischen den beiden Bügeln 4 und 5 sind aus distaler Richtung offene Spalten vorhanden, in die Teile der Herzklappenprothese eingeführt und dort fixiert wedren können.

Der hier äußere Bügel 5 ist in proximaler Richtung weiter nach innen gezogen und mit seinem Ende mit jeweils einem Stützsteg 8 verbunden. Die Stützstege 8 sind bei diesem Beispiel parallel zur Längsachse der Vorrichtung ausgerichtet und sie bilden gemeinsam mit sägezahn-, rauten- oder mäanderförmigen Querstegen einen Stützkörper, der im entfalteten Zustand an der Aortenwand anliegt. Zur Verhakung können zusätzliche Spitzen 9 an den Stützstegen 8 und/oder den Querstegen vorhanden bzw. ausgebildet sein, die sich in der Aortenwand verhaken.

Die Anordnung und Länge der Stützstege 8 und der entsprechend große Abstand zur im Bereich der Bügel 3,4 und 5 befestigten Herzklappenprothese, ermöglichen das Positionieren der Herzklappenprothese ohne die Herzkranzgefäße zu verschließen bzw. abzudecken.

Bei dem hier gezeigten Beispiel sind zwischen den einzelnen in 120 ° Anordnung verwendeten Segmenten zusätzliche proximal eingezogene Bügel 2 vorhanden, die mit den distal äußeren Bügeln 5 verbunden sind. Auch hierbei sind die Verbindungen Festkörpergelenke 6, die jedoch möglichst in einem Abstand zu den Festkörpergelenken 7 angeordnet sein sollten. Es können so zwei Hebel pro Segment genutzt und mit einem solchen doppelt gespiegelten Aufbau in etwa doppelt große Kräfte realisiert werden, um die Vorrichtung zu fixieren.

Im entfalteten implantierten Zustand können zwischen den Teilen 1 der Bügel 5 und den Bügeln 2 wiederum Teile der Herzklappenprothese wechselweise eingeführt, so gestützt und daran befestigt werden.

Die Anzahl der verwendeten Bügel kann aber noch erhöht werden, um den Halt zu verbessern und die Belastung der Herzklappenprothese weiter zu verringern.

## Patentansprüche

1. Vorrichtung zur Befestigung und Verankerung von Herzklappenprothesen, die aus drahtförmigen Elementen gebildet und radial symmetrisch ausgebildet ist, **dadurch gekennzeichnet, dass** an der Vorrichtung zur Befestigung und Abstützung einer Herzklappenprothesedrei jeweils gleiche Paare mit einem ersten distal angeordneten und gekrümmten Bügel (4) und einem entgegengesetzt gekrümmten Bügel (3) in jeweils um 120 ° versetzter Anordnung miteinander mittels Festkörpergelenken (7) verbunden sind und die Festkörpergelenke (7) die Funktion von Drehlagern erfüllen.

2. Vorrichtung nach Anspruch 1, **dadurch gekennzeichnet, dass** die beiden zueinander entgegengesetzt gebogenen Bügel (3, 4) in Bezug zu den Festkörpergelenken (7) gleich lange Hebelarme bilden.

3. Vorrichtung nach Anspruch 1 oder 2, **dadurch gekennzeichnet, dass** die Herzklappenprothese an dem ersten aügel (4) und/oder einem zweiten distalen Bügel (5) befestigt ist.

4. Vorrichtung nach einem der Ansprüche 1 bis 3, **dadurch gekennzeichnet, dass** der erste und zweite distale Bügel (4, 5) teilweise in gleicher Richtung gebogen sind und parallel zueinander verlaufen, so dass dazwischen Spalte zur Einführung von Teilen der Herzklappenprothese ausgebildet sind.

5. Vorrichtung nach einem der Ansprüche 1 bis 4, **dadurch gekennzeichnet, dass** am zweiten distalen Bügel proximal eingezogene Teile (1) vorhanden sind, an denen weitere Bügel (2) mit Festkörpergelenken (6) befestigt sind.

6. Vorrichtung nach einem der Ansprüche 1 bis 5, **dadurch gekennzeichnet, dass** die Festkörpergelenke (6) und (7) in einem Abstand zueinander angeordnet sind.

7. Vorrichtung nach einem der Ansprüche 1 bis 6, **dadurch gekennzeichnet, dass** die proximalen Enden des ersten Bügels (4) oder des Teiles (1) des zweiten Bügels (5) mit parallel zur Längsachse ausgerichteten Stützstegen (8), die in Verbindung mit einem sägezahn-, rauten- oder mäanderförmigen Drahtgebilde einen auffaltbaren Stützkörper bilden, verbunden sind.

8. Vorrichtung nach einem der Ansprüche 1 bis 7, **dadurch gekennzeichnet, dass** am Stützkörper Spitzen (9) zur Verankerung in der Aortenwand ausgebildet sind.

9. Vorrichtung nach einem der Ansprüche 1 bis 8, **dadurch gekennzeichnet, dass** sämtliche Teile (1 bis 9) aus einem Formgedächtnismetall gebildet sind.

## Claims

1. Device for fastening and anchoring of cardiac valve prostheses, which is formed from wire-shaped elements and disposed radially symmetrically **characterized in that** on the device for fastening and support of a cardiac valve prosthesis three identical pairs of hoops with a first distally arranged and curved hoop (4) and an opposed curved hoop (3) are each connected together in an arrangement displaced by 120° by solid articulations (7) and **in that** said solid articulations (7) fulfill the function of pivots.

2. Device in accordance with Claim 1, **characterized in that** the two curved hoops (3, 4) opposed to one another form in respect of the solid articulations (7) lever arms of equal length.

3. Device in accordance with Claim 1 or 2, **characterized in that** the cardiac valve prosthesis is fastened on the first hoop (4) and/or a second distal hoop (5).

4. Device in accordance with one of the Claims 1 to 3, **characterized in that** the first and the second distal hoops (4, 5) are partly bent in the same direction and run parallel to one another, so that caps of formed in between them for insertion of parts of the cardiac valve prosthesis.

5. Device in accordance with one of the Claims 1 to 4, **characterized in that** proximally retracted parts (1) are provided on the second distal hoop, on which further hoops (2) with solid articulations (6) are fastened.

6. Device in accordance with one of the Claims 1 to 5, **characterized in that** the solid articulations (6) and (7) are arranged at a distance from one another.

7. Device in accordance with one of the Claims 1 to 6, **characterized in that** the proximal ends of the first hoop (4) or proximally retracted parts (1) provided on the second hoop (5) are connected to one another by means of support webs (8), oriented parallel to the longitudinal axis, which form a foldable support body in combination with a saw tooth, rhombic, or wave-like wire formation.

8. Device in accordance with one of the Claims 1 to 7, **characterized in that** barbs (9) are disposed on the support body for anchoring in the wall of the aorta.

9. Device in accordance with one of the Claims 1 to 8, **characterized in that** all parts (1 to 9) are formed from a shape memory metal.

## Revendications

1. Dispositif pour fixer et ancrer des prothèses de valvules cardiaques, qui est formé par des éléments de forme filaire et réalisé à symétrie radiale,
**caractérisé en ce que** sur le dispositif pour fixer et soutenir une prothèse de valvule cardiaque, trois paires respectives identiques pourvues d'un premier étrier agencé distal et recourbé (4) et d'un étrier (3) recourbé en sens opposé sont reliées entre elles en une disposition décalée de 120° au moyen d'articulations à corps solide (7) et les articulations à corps solide (7) remplissent la fonction de paliers rotatifs.

2. Dispositif selon la revendication 1, **caractérisé en ce que** les deux étriers (3, 4) cintrés en sens opposés l'un à l'autre forment des bras de levier de même longueur par rapport aux articulations à corps solide (7).

3. Dispositif selon la revendication 1 ou 2, **caractérisé en ce que** la prothèse de valvule cardiaque est fixée sur le premier étrier (4) et/ou sur un second étrier distal (5).

4. Dispositif selon l'une des revendications 1 à 3, **caractérisé en ce que** le premier et le second étrier distal (4, 5) sont cintrés partiellement dans le même sens et s'étendent parallèlement l'un à l'autre, de sorte que des intervalles y sont formés pour introduire des parties de la prothèse de valvule cardiaque.

5. Dispositif selon l'une des revendications 1 à 4, **caractérisé en ce que** sur le second étrier distal sont prévues des parties (1) en retrait proximal sur lesquelles sont fixés d'autres étriers (2) avec des articulations à corps solide (6).

6. Dispositif selon l'une des revendications 1 à 5, **caractérisé en ce que** les articulations à corps solide (6) et (7) sont agencées à distance l'une de l'autre.

7. Dispositif selon l'une des revendications 1 à 6, **caractérisé en ce que** les extrémités proximales du premier étrier (4) ou de la partie (1) du second étrier (5) sont reliées à des barrettes de soutien (8) orientées parallèlement à l'axe longitudinal et constituant un corps de soutien dépliable en association avec un ensemble construit de fil sous forme de dents de scie, de losanges ou de méandres.

8. Dispositif selon l'une des revendications 1 à 7, **caractérisé en ce que** sur le corps de soutien sont réalisées des pointes (9) pour l'ancrage dans la paroi aortique.

9. Dispositif selon l'une des revendications 1 à 8, **caractérisé en ce** toutes les parties (1 à 9) sont constituées en un métal à mémoire de forme.
